# EUROPEAN PATENT APPLICATION

(11) **EP 4 099 723 A1**
(43) Date of publication of application: **07.12.2022**
(21) Application number: 22175340.3
(22) Date of filing: 25.05.2022
(51) Int. Cl.: H04R 25/00, A61B 17/86, A61F 2/30

(54) **AN ANCHORING FIXTURE WITH IMPROVED CUTTING CAPABILITY**

(30) Priority: 01.06.2021 EP 21177009
(71) Applicant: Oticon Medical A/S, 2765 Smørum (DK)
(72) Inventor: JOHANSSON, Martin, 43632 Askim (SE)
(74) Representative: Demant

(57) **Abstract**

An anchoring fixture for anchoring a prothesis to a skull bone may comprise a screw thread apparatus including a screw thread having a varying outer diameter and formed along a longitudinal length of the anchoring fixture; a flange configured to function as a stop for the anchoring fixture adapted to rest on top of the bone when the anchoring fixture is implanted into the bone; at least a main relief chamber formed into a portion of the screw thread having a first side along the longitudinal length that is curved shaped, and wherein the anchoring fixture is tapered over at least a first portion of the longitudinal length of the anchoring fixture and where the first portion is arranged at a distal end of the anchoring fixture, and where the distal end is opposite to the flange.

## Description

### FIELD OF THE DISCLOSURE

The present disclosure relates to an anchoring fixture. More particularly, the disclosure relates to an anchoring fixture with improved cutting capability.

### BACKGROUND

Medical implants such as bone anchored hearing aid systems are applied for the rehabilitation of patients suffering from hearing losses for which traditional hearing aids are insufficient. A typical bone anchored hearing aid system comprises an external hearing aid provided with a vibrating transducer connected to a skin-penetrating abutment through a coupling. The abutment may have an interconnection to an implantable anchoring fixture that is configured to anchor a bone anchored hearing aid system the skull bone of the patient. The anchoring fixture is typically made of titanium and may be provided with a flange to prevent the fixture from being pushed through the skull bone when exposed to a sudden accidental impact.

The current anchoring fixtures that are used are 3 and 4 mm long. Depending on the bone thickness of the patient, either a 3 or 4 mm implant is installed. Therefore, a 4 mm deep osteotomy is initially created to accommodate a 3 mm fixture. After confirmation of bone in the bottom of the osteotomy, the hole may be deepened to 5 mm to accommodate a 4 mm long implant. That depth would exclude children and other patients with non-normal anatomy. Hence it would be beneficial to only drill down to 4 mm for installation of a shorter implant.

The available bone thickness at the site of implantation for an individual patient differs and especially for young children there might not be enough bone for a 4 mm implant (5 mm drilling depth) or even a 3 mm implant. In addition, the bone thickness is commonly not evaluated prior surgery with techniques, such as MRI or CT.

A central aspect of the bone anchored hearing implant, or any implant installed in the bone, is the primary stability (stability of the implant just after surgery) and secondary stability (stability of the implant after osseointegration). The primary stability may be considered a fundamental prerequisite to acquiring osseointegration. It is considered highly desirable that this level of stability should be as high as possible. However, in systems inducing a high level of stability for example by use of a tapered implant or a drilling sequence with a drill size much smaller than the implant diameter, might result in over-compression of the bone. It is clear therefore that there is a very complex and subtle inter-relationship between bone quality, stability, geometry and placement technique.

Therefore, there is a need for shorten the anchoring fixture such that it is possible to install it in a hole of max 4mm depth, while keeping the bone to implant contact as high as possible, without generating too high insertion torque and bone compression. In addition, improved geometry and increased manufacturability is also an objective.

### SUMMARY

According to an aspect of the present disclosure, an anchoring fixture is disclosed. The anchoring fixture for anchoring a prothesis to a skull bone may comprise a screw thread apparatus including a screw thread having a varying outer diameter and formed along a longitudinal length of the anchoring fixture; a flange configured to function as a stop for the anchoring fixture adapted to rest on top of the bone when the anchoring fixture is implanted into the bone; at least a main relief chamber formed into a portion of the screw thread having a first side along the longitudinal length that is curved shaped, and wherein the anchoring fixture is tapered over at least a first portion of the longitudinal length of the anchoring fixture and where the first portion is arranged at a distal end of the anchoring fixture, and where the distal end is opposite to the flange.

The first side of the main relief chamber may be helix shaped.

By having a curved shaped relief chamber results in an increased cutting capability of the anchoring fixture. Furthermore, by specifying the curved shaped to be helix shaped has shown that it has a surprisingly good effect on the cutting capability.

The main relief chamber may have a main relief length that extends from the distal end of the anchoring fixture and along the longitudinal length. The main relief length may be between 2 mm and 3 mm or 2.5 mm and 2.8 mm.

The anchoring fixture may comprise at least three main relief chambers arranged at least partially symmetrically around a center of the anchoring fixture, and wherein each of the at least three main relief chambers is formed into a portion of the screw thread having a first side along the longitudinal length that is curved shaped. Thereby, the cutting capability is improved even more.

An opening angle of the main relief chamber may be above 50 degrees.

The at least three main relief chambers may be arranged around a center of the anchoring fixture and wherein a circumferential distance between at least two of the at least three main relief chambers may be shorter than other circumferential distances between the at least three main relief chambers, thereby the opening angle of at least one of the at least two will have an opening angle that is lower than the opening angle of the other main relief chambers.

An opening of one or more of the at least three main relief chambers may be below 60 degrees for the purpose of improving the cutting capability of the fixture.

The anchoring fixture may comprise at least three main relief chambers arranged around a center of the anchoring fixture, and wherein each of the at least three main relief chambers may be formed into a portion of the screw thread having a first side along the longitudinal length that is curved shaped. The anchoring fixture may comprise a plurality of sub cutting relief chambers formed into a portion of the screw thread, and wherein the plurality of sub cutting relief chambers are formed between at least two of the at least three main relief chambers. Thereby, the cutting capability is even more improved.

A sub relief thickness of each of the plurality of sub cutting relief chambers along the longitudinal length of the anchoring fixture may not be larger than a thread thickness of a single thread of the screw thread apparatus along the longitudinal length. The sub relief chambers may be formed on the thread and not between two threads.

The anchoring fixture may be tapered over at least a second portion of the longitudinal length of the anchoring fixture, and where the tapering of the second portion may be in reverse in relation to the tapering of the first portion along the longitudinal length, and where the second portion may be arranged in vicinity to the flange and distal to the first portion of the anchoring fixture. By tapering the threads in both the first portion and/or the second portion results in a decrease of the insertion force while implanting the fixture. By reverse tapering the second portion in relation to the tapering of the first portion results in an even more decreased insertion force.

A length of the anchoring fixture along the longitudinal length and from a bottom of the flange to the distal end of the anchoring fixture is no greater than 4 mm.

A first tapered length of the tapered first portion along the longitudinal length of the anchoring fixture may be between 0.65 mm and 1.1 mm or 0.7 mm and 1 mm. Having a length of 1 mm has shown as being the ideal length for obtaining an optimal insertion force.

A cone angle of the tapered first portion may be between 26 and 36 degrees. Having a cone angle of around 35 degrees or 26 degrees have shown as being the ideal angle for obtaining an optimal insertion force.

A cone angle of the tapered first portion may be of above 35 degrees.

A bottom surface of the anchoring fixture arranged at the distal end may have a conical shape configured such that the anchoring fixture is a self-drilling fixture.

For improving the self-drilling capability, the conical shape bottom surface may include a plurality of bottom relief chambers.

Aforementioned features shall be considered to be disclosed in any combination with each other. Further, the disclosure of any means for performing a method step shall be understood to also disclose the respective method step and the disclosure of a method step shall be understood to also disclose respective means for performing the step.

### BRIEF DESCRIPTION OF DRAWINGS

The aspects of the disclosure may be best understood from the following detailed description taken in conjunction with the accompanying figures. The figures are schematic and simplified for clarity, and they just show details to improve the understanding of the claims, while other details are left out. Throughout, the same reference numerals are used for identical or corresponding parts. The individual features of each aspect may each be combined with any or all features of the other aspects. These and other aspects, features and/or technical effect will be apparent from and elucidated with reference to the illustrations described hereinafter in which:
FIG. 1 illustrates an example of an anchoring fixture;
FIG. 2 illustrates an example of an anchoring fixture;
FIG. 3 illustrates an example of an anchoring fixture;
FIG. 4 illustrates an example of an anchoring fixture;
FIG. 5 illustrates an example of an anchoring fixture; and
FIG. 6 illustrates measured insertion torque for three different configurations of a anchoring fixture.

### DETAILED DESCRIPTION

The detailed description set forth below in connection with the appended drawings is intended as a description of various configurations. The detailed description includes specific details for the purpose of providing a thorough understanding of various concepts. However, it will be apparent to those skilled in the art that these concepts may be practiced without these specific details. Several aspects of the apparatus and methods are described by various blocks, functional units, modules, components, circuits, steps, processes, algorithms, etc. (collectively referred to as "elements"). Depending upon particular application, design constraints or other reasons, these elements may be implemented using electronic hardware, computer program, or any combination thereof.

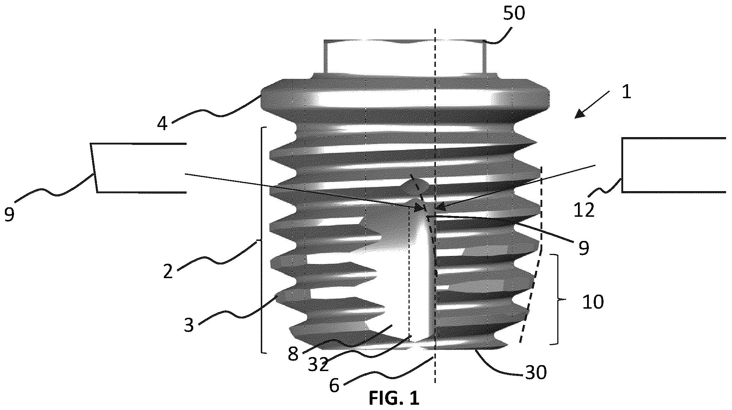

FIG. 1 is illustrating an anchoring fixture 1 for anchoring a prothesis to a skull bone. The fixture 1may comprise a screw thread apparatus 2 including a screw thread 3 having a varying outer diameter and formed along a longitudinal length 6 of the anchoring fixture 1. The fixture 1 includes a flange 4 configured to function as a stop for the anchoring fixture 1 adapted to rest on top of the bone when the anchoring fixture 1 is implanted into the bone. A main relief chamber 8 is formed into a portion of the screw thread 3 having a first side 9 along the longitudinal length 6 that is curved shaped, and wherein the anchoring fixture 1 is tapered over at least a first portion 10 of the longitudinal length 6 of the anchoring fixture 1 and where the first portion 10 is arranged at a distal end 30 of the anchoring fixture 1, and where the distal end 30 is opposite to the flange 4. The main relief chamber 8 has a main relief length 32 that extends from the distal end 30 of the anchoring fixture 1 and along the longitudinal length 6.

The main relief length 32 is between 2 mm and 3 mm or 2.5 mm and 2.8 mm.

As seen from the FIG. when having a curved shaped, i.e. a helix shaped, the cutting capability is improved as the cutting edge 11 becomes more angled in comparison to a straight 12 first side 9.

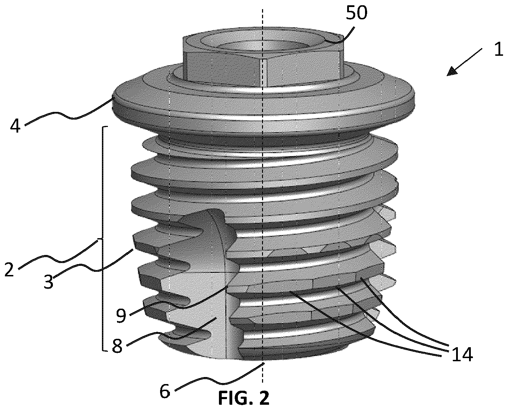

FIG. 2 illustrates a further example of the anchoring fixture 1 including a plurality of sub cutting relief chambers 14 formed into a portion of the screw thread 3, and wherein the plurality of sub cutting relief chambers 14 are formed between at least two main relief chambers 8. A sub relief thickness of each of the plurality of sub cutting relief chambers 14 along the longitudinal length 6 of the anchoring fixture is not larger than a thread thickness of a single thread 3 of the screw thread apparatus 2 along the longitudinal length.

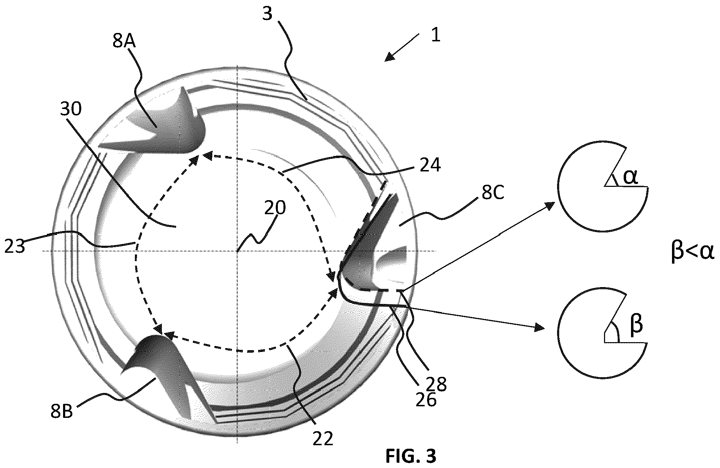

FIG. 3 illustrates a further example of the anchoring fixture 1 including at least three main relief chambers (8A,8B,8C) arranged at least partially symmetrically around a center 20 of the anchoring fixture 1, and wherein each of the at least three main relief chambers (8A,8B,8C) is formed into a portion of the screw thread 3 having a first side 9 along the longitudinal length that is curved shaped. Further in FIG. 3, a circumferential distance 22 between at least two (8B,8C) of the at least three main relief chambers (8A,8B,8C) is shorter than other circumferential distances (23,24) between the at least three main relief chambers (8A,8B,8C).

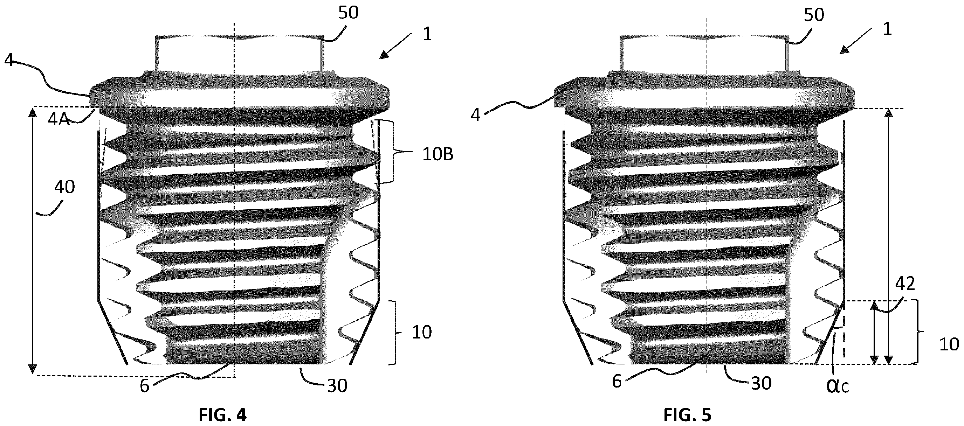

FIG. 4 illustrates an example of an anchoring fixture 1, wherein the anchoring fixture 1 is tapered over at least a second portion 10B of the longitudinal length 6 of the anchoring fixture 1, and where the tapering of the second portion 10B is in reverse in relation to the tapering of the first portion 10 along the longitudinal length 6, and where the second portion 10B is arranged in vicinity to the flange 4 and distal to the first portion 10 of the anchoring fixture 1. A length 40 of the anchoring fixture 1 along the longitudinal length 6 and from a bottom 4A of the flange 4 to the distal end 30 of the anchoring fixture 1 is no greater than 4 mm.

FIG. 5 illustrates an example of an anchoring fixture 1 having a cone angle (α_{c}) at the tapered first portion 10 of between 26 and 36 degrees. In another example, the cone angle (α_{c}) of the tapered first portion 10 is above 35 degrees. A first tapered length 42 of the tapered first portion 10 along the longitudinal length 6 of the anchoring fixture 1 is between 0.65 mm and 1.1 mm or 0.7 mm and 1 mm.

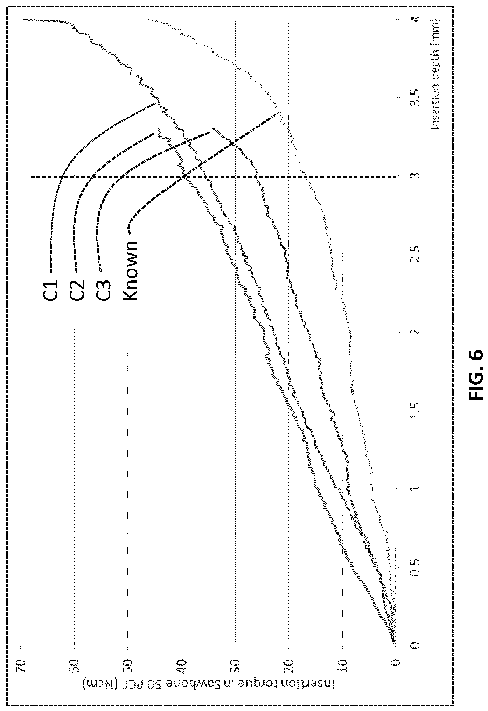

FIG. 6 illustrates measured insertion torque for three different configurations (C1,C2,C3) of the anchoring fixture 1 in comparison to a known fixture design (known). C1 implies a length 40 of the anchoring fixture 1 of 4 mm, a length 42 of the first portion 10 of 1mm and a cone angle (α_{c}) of about 26 degrees. C2 implies a length 40 of the anchoring fixture 1 of about 3.3 mm, a length 42 of the first portion 10 of 1mm and a cone angle (α_{c}) of about 26 degrees. C3 implies a length 40 of the anchoring fixture 1 of about 3.3 mm, a length 42 of the first portion 10 of 0.7mm and a cone angle (α_{c}) of about 35 degrees. The results suggests that a cone angle of between 25 degrees and 35 degrees is optimal for obtaining an optimal insertion torque.

In all examples of the anchoring fixture 1, the anchoring fixture 1 includes a connection interface 50 to a skin-penetrating abutment. However, the connection interface 50 is not essential for the disclosure, and thereby, not mentioned in the claims.

The anchoring fixture 1 may have a bottom surface 30 that has a conical shape that results in a self-drilling fixture. The conical shape bottom surface 30 may include a plurality of bottom relief chambers for the purpose of improving the drilling capability of the fixture 1.

It is intended that the structural features of the devices described above, either in the detailed description and/or in the claims, may be combined with steps of the method, when appropriately substituted by a corresponding process.

As used, the singular forms "a," "an," and "the" are intended to include the plural forms as well (i.e. to have the meaning "at least one"), unless expressly stated otherwise. It will be further understood that the terms "includes," "comprises," "including," and/or "comprising," when used in this specification, specify the presence of stated features, integers, steps, operations, elements, and/or components, but do not preclude the presence or addition of one or more other features, integers, steps, operations, elements, components, and/or groups thereof. It will also be understood that when an element is referred to as being "connected" or "coupled" to another element, it can be directly connected or coupled to the other element, but an intervening element may also be present, unless expressly stated otherwise. Furthermore, "connected" or "coupled" as used herein may include wirelessly connected or coupled. As used herein, the term "and/or" includes any and all combinations of one or more of the associated listed items. The steps of any disclosed method are not limited to the exact order stated herein, unless expressly stated otherwise.

It should be appreciated that reference throughout this specification to "one embodiment" or "an embodiment" or "an aspect" or features included as "may" means that a particular feature, structure or characteristic described in connection with the embodiment is included in at least one embodiment of the disclosure. Furthermore, the particular features, structures or characteristics may be combined as suitable in one or more embodiments of the disclosure.

The previous description is provided to enable any person skilled in the art to practice the various aspects described herein. Various modifications to these aspects will be readily apparent to those skilled in the art, and the generic principles defined herein may be applied to other aspects. Reference to an element in the singular is not intended to mean "one and only one" unless specifically so stated, but rather "one or more." Unless specifically stated otherwise, the term "some" refers to one or more.

Accordingly, the scope should be judged in terms of the claims that follow.

## Claims

1. An anchoring fixture for anchoring a prothesis to a skull bone, comprising:
• a screw thread apparatus including a screw thread having a varying outer diameter and formed along a longitudinal length of the anchoring fixture;
• a flange configured to function as a stop for the anchoring fixture adapted to rest on top of the bone when the anchoring fixture is implanted into the bone;
• at least a main relief chamber formed into a portion of the screw thread having a first side along the longitudinal length that is curved shaped, and
wherein the anchoring fixture is tapered over at least a first portion of the longitudinal length of the anchoring fixture and where the first portion is arranged at a distal end of the anchoring fixture, and where the distal end is opposite to the flange.

2. An anchoring fixture according to claim 1, wherein the first side of the main relief chamber is helix shaped.

3. An anchoring fixture according to any of the previous claims, wherein the main relief chamber has a main relief length that extends from the distal end of the anchoring fixture and along the longitudinal length.

4. An anchoring fixture according to claim 3, wherein the main relief length is between 2 mm and 3 mm or 2.5 mm and 2.8 mm.

5. An anchoring fixture according to any of the previous claims, comprising at least three main relief chambers arranged at least partially symmetrically around a center of the anchoring fixture, and wherein each of the at least three main relief chambers is formed into a portion of the screw thread having a first side along the longitudinal length that is curved shaped.

6. An anchoring fixture according to any of claims 1 to 4, comprising at least three main relief chambers arranged around a center of the anchoring fixture and wherein a circumferential distance between at least two of the at least three main relief chambers is shorter than other circumferential distances between the at least three main relief chambers, and wherein each of the at least three main relief chambers is formed into a portion of the screw thread having a first side along the longitudinal length that is curved shaped.

7. An anchoring fixture according to any of the previous claims, comprising at least three main relief chambers arranged around a center of the anchoring fixture, and wherein each of the at least three main relief chambers is formed into a portion of the screw thread having a first side along the longitudinal length that is curved shaped, and the anchoring fixture comprises a plurality of sub cutting relief chambers formed into a portion of the screw thread, and wherein the plurality of sub cutting relief chambers are formed between at least two of the at least three main relief chambers.

8. An anchoring fixture according to claim 7, wherein a sub relief thickness of each of the plurality of sub cutting relief chambers along the longitudinal length of the anchoring fixture is not larger than a thread thickness of a single thread of the screw thread apparatus along the longitudinal length.

9. An anchoring fixture according to any of the previous claims, wherein the anchoring fixture is tapered over at least a second portion of the longitudinal length of the anchoring fixture, and where the tapering of the second portion is in reverse in relation to the tapering of the first portion along the longitudinal length, and where the second portion is arranged in vicinity to the flange and distal to the first portion of the anchoring fixture.

10. An anchoring fixture according to any of the previous claims, wherein a length of the anchoring fixture along the longitudinal length and from a bottom of the flange to the distal end of the anchoring fixture is no greater than 4 mm.

11. An anchoring fixture according to any of the previous claims, wherein a first tapered length of the tapered first portion along the longitudinal length of the anchoring fixture is between 0.65 mm and 1.1 mm or 0.7 mm and 1 mm.

12. An anchoring fixture according to any of the previous claims, wherein a cone angle of the tapered first portion is between 26 and 36 degrees.

13. An anchoring fixture according to any of claims 1 to 11, wherein a cone angle of the tapered first portion is above 35 degrees.

14. An anchoring fixture according to any of the previous claims, wherein a bottom surface of the anchoring fixture arranged at the distal end is having a conical shape configured such that the anchoring fixture is a self-drilling fixture.

15. An anchoring fixture according to claim 14, wherein the conical shape bottom surface includes a plurality of bottom relief chambers.
